# EUROPÄISCHE PATENTSCHRIFT

(11) **EP 2 710 961 B1**
(45) Veröffentlichungstag und Bekanntmachung des Hinweises auf die Patenterteilung: **07.08.2019**
(21) Anmeldenummer: 12185733.8
(22) Anmeldetag: 24.09.2012
(51) Int. Cl.: A61B 8/06, A61B 8/08, A61B 8/00, A61B 8/04, A61B 5/022

(54) **Druckmessvorrichtung zur Druckmessung einer Vene oder eines Organs und zur Kombination mit einer Ultraschallmesseinheit, sowie System und Verfahren zur Venen-/Organdruckmessung**
Pressure measurement device for pressure measurement of a vein or an organ and for combination with an ultrasound measuring unit, and system and method for venous/organ pressure measurement
Dispositif de mesure de pression destiné à mesurer la pression d'une veine ou d'un organe et destiné à se combiner à une unité de mesure par ultrasons, ainsi que système et procédé de mesure de la pression d'une veine/d'un organe

(43) Veröffentlichungstag der Anmeldung: 26.03.2014
(73) Patentinhaber: Veinpress GmbH, 3110 Münsingen (CH)
(72) Erfinder: Baumann, Ulrich, 3110 Münsingen (CH)
(74) Vertreter: Patentbüro Paul Rosenich AG

(56) Entgegenhaltungen:
- EP-A2- 1 522 258
- WO-A1-01/00087
- WO-A1-85/00278
- US-A- 4 926 874
- US-A1- 2012 041 313

## Beschreibung

Die Erfindung bezieht sich auf eine Druckmessvorrichtung nach dem Oberbegriff des Anspruchs 1, sowie ein System zur Druckmessung von Venen oder Organen gemäss Anspruch 12 und ein Verfahren zur Venendruckmessung gemäss Anspruch 13. Hierbei soll der Druck im Gewebe, vorzugsweise an einer Extremität bzw. eines Körperteils eines Lebewesens, gemessen werden, wobei gleichzeitig das Gewebe mittels Ultraschall und dessen Reflexion am Gewebe auswertenden Verfahren beobachtet wird.

Generell sind Ultraschalluntersuchungen mit Hilfe kommerziell erhältlicher Ultraschallmesseinheiten, insbesondere Schallköpfen und bildgebenden bzw. bildverarbeitenden Verfahren aus dem medizinischen Untersuchungsalltag bekannt. Dabei wird von der Ultraschallmesseinheit Ultraschall in das Gewebe eingestrahlt, dort reflektiert und die Laufzeitunterschiede werden durch bildgebende Verfahren zu einem Bild vom Inneren des zu untersuchenden Gewebes verarbeitet. Hierbei ist die Anwendung eines Druckes auf die Ultraschallmesseinheit zur Charakterisierung von druckbedingten Gewebe- und Gefässveränderungen gut etabliert. Insbesondere wird dies bei der Venenkompression zur Thrombosediagnostik angewendet. Im Falle dieser Untersuchungsverfahren hängt jedoch der ausgeübte Druck von der Erfahrung des Bedieners der Messvorrichtung ab und ist von Untersucher zu Untersucher unterschiedlich. Im Zusammenhang mit der Darstellung von Gefässstrukturen und sich darin bewegenden Körperflüssigkeiten werden zur Bestimmung der Strömungsgeschwindigkeit insbesondere Dopplerverfahren eingesetzt, die eine Zusatzausrüstung zu kommerziell erhältlichen Ultraschallgeräten bedingen.

Aus der US 6,086,533 A ist bekannt, einer Ultraschallmesseinheit ein Behältnis in Form einer Blase gefüllt mit einer ultraschalltransparenten bzw. ultraschalldurchlässigen Flüssigkeit vorzuordnen, um den durch den Schallkopf aufgebrachten Druck auf ein Gewebe messen zu können. Dazu wird mittels der Ultraschallmesseinheit Druck auf die vorangeordnete Blase ausgeübt, welche diesen Druck an das darunterliegende Gewebe weitergibt. Somit lässt sich die Kraft bestimmen, die ein Untersucher auf eine darunterliegende Gewebeoberfläche ausübt. Ferner wird mittels eines Dopplerverfahrens die Geschwindigkeit der im Gefäss strömenden Körperflüssigkeit gemessen. Im Anschluss lässt sich aus der von aussen aufgebrachten Kraft und mittels der Strömungsgeschwindigkeit der Flüssigkeitsdruck im Gefäss bestimmen.

Praktische Versuche mit derartigen Anordnungen haben jedoch gezeigt, dass die räumliche Auflösung von den in der Medizin üblichen Ultraschallmesseinheiten durch das Vorschalten einer derartigen Druckmessvorrichtung regelmässig stark beeinträchtigt bis unbrauchbar wird. Die US 6,086,533 A lehrt ferner, dass der Druck in der Blase mittels eines Druckregulators regelbar ist. Demgemäss kann der Druck, der durch die Kontaktoberfläche der Blase aufgebracht wird, gesteuert werden oder ein automatisiertes oder vorgeschriebenes Protokoll von anzuwendenden Drücken kann abgearbeitet werden.

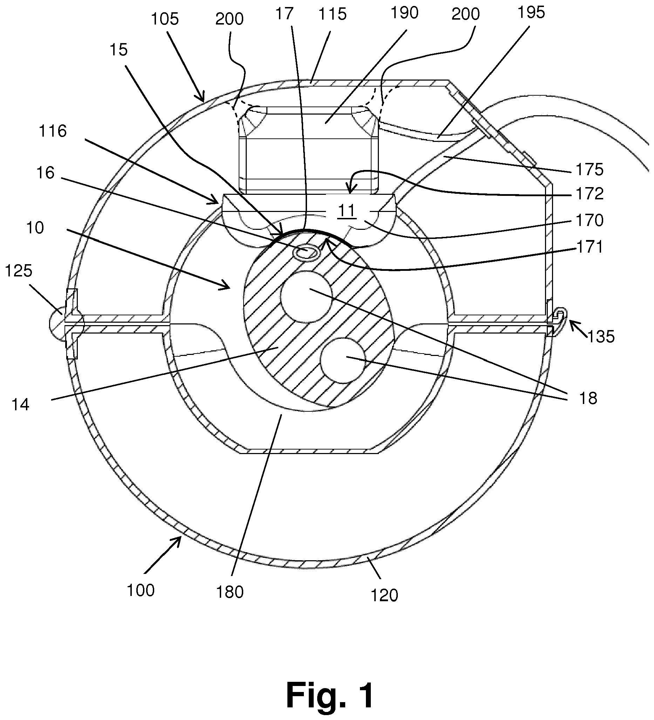

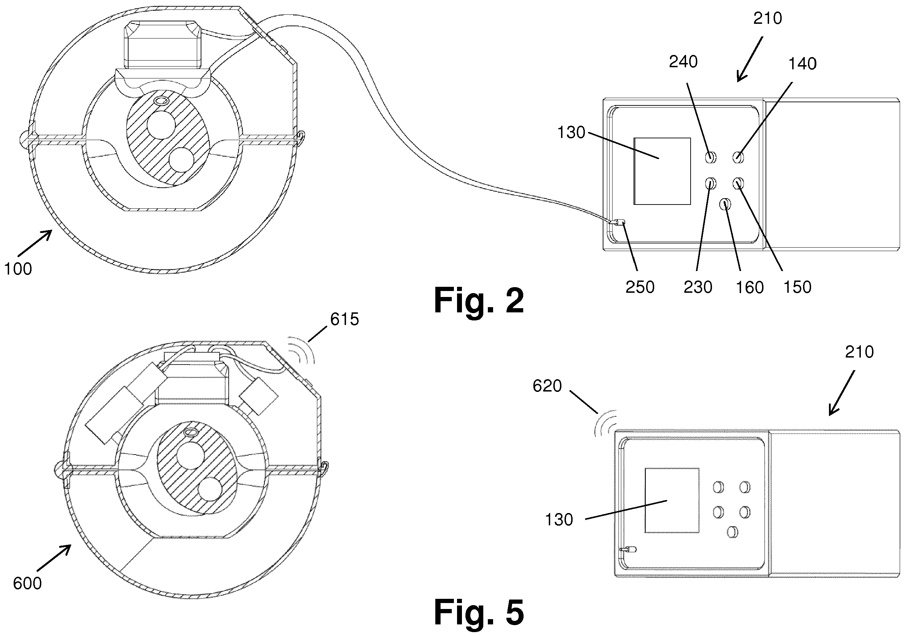

Ferner ist aus der Veröffentlichung EP 1 415 596 A1 eine Druckmessvorrichtung für eine Ultraschallmesseinheit bekannt. Die dort beschriebene Druckmessvorrichtung besteht im Wesentlichen aus einem starren Behältnis, zum Beispiel in Form eines flachen Hohlzylinders. An einer Flachseite ist eine steife Membran eingelassen, die der Ankopplung an den Ultraschallmesskopf dient. An der gegenüberliegenden Flachseite ist eine flexible Auflagemembran angebracht, welche den vom Gehäuse gebildeten Hohlraum abschliesst. Der Innenraum der Druckmessvorrichtung ist mit einer ultraschalltransparenten Flüssigkeit möglichst vollständig und blasenfrei gefüllt. Wie aus Figur 2 der EP 1 415 596 A1 erkennbar ist, befindet sich ein Ansatzstutzen am Gehäuse, über welchen dieses mit einem Flüssigkeitsreservoir in Verbindung gebracht werden kann. Erkennbar ist eine Leitung in Figur 1, mit welcher der in dem Gehäuse (Druckmesskapsel) vorhandene Druck mittels einer externen Apparatur bestimmt werden kann.

Insbesondere bei Notfällen besteht oftmals ein Bedürfnis eine Druckmessvorrichtung an einem Körperteil, insbesondere an einer Extremität eines Lebewesens, derart anzuordnen, dass eine einwandfreie Messung mit dieser Druckmessvorrichtung auch in Stresssituationen für den Untersucher erfolgen kann. Beispielsweise aus der EP 2 386 249 A1 ist eine Ultraschalldiagnosevorrichtung bekannt, welche eine bandförmige Haltevorrichtung zur Festlegung an dem zu diagnostizierenden Objekt umfasst. Diese bekannte Ultraschalldiagnosevorrichtung ist jedoch für eine Druckmessvorrichtung zur Druckmessung von Venen oder Organen nicht geeignet.

Aus der US 2011/0196238 A1 ist eine gattungsgemässe Druckmessvorrichtung zur Kombination mit einer Ultraschallmesseinheit bekannt, die einen Gehäuseabschnitt, einen Festlegeabschnitt zum Festlegen der Druckmessvorrichtung an einem Körperteil und ein an dem Gehäuseabschnitt vorgesehenes, flexibles Behältnis aufweist. Das Behältnis ist mit einer ultraschalltransparenten Flüssigkeit gefüllt und aus einem ultraschalldurchlässigen Material gefertigt. Das Behältnis ist so ausgestaltet, dass ein von der Ultraschallmesseinheit ausgehender Ultraschall durch dieses durchtritt sowie vom Gewebe reflektierter Ultraschall ebenso durch dieses durchtritt. Die Auflageseite des Behältnisses ist perforiert, damit die ultraschalltransparente Flüssigkeit an der Oberfläche des Gewebes des Körperteils unter Druckausübung auf das Behältnis austreten kann.

Weitere Druckmessvorrichtungen sind aus WO85/00278A1 sowie EP1522258A2 bekannt.

Auch diese bekannte Ultraschalldiagnosevorrichtung ist für eine Druckmessung von Venen oder Organen nicht geeignet.

Aufgabe der vorliegenden Erfindung ist es somit, eine Druckmessvorrichtung sowie ein Druckmesssystem sowie ein Verfahren zur Druckmessung von Venen und Organen zu schaffen, welche bzw. welches die vorgenannten Nachteile nicht aufweist und insbesondere weniger störanfällig und einfacher zu bedienen sind als bisherige Lösungen.

Die Aufgabe wird für eine Druckmessvorrichtung zur Venen- und/oder Organdruckmessung gemäss den Merkmalen des Patentanspruches 1 und für ein Druckmesssystem zur Venen- und/oder Organdruckmessung gemäss den Merkmalen des Patentanspruches 12 gelöst, während der Patentanspruch 13 eine Lösung für ein Verfahren zur Venen- und/oder Organdruckmessung angibt.

Vorteilhafte Weiterbildungen sind in den Figuren und in den von den vorgenannten Ansprüchen abhängigen Patentansprüchen dargelegt.

Gemäss der Erfindung nach Anspruch 1 umschliesst das Behältnis ein abgeschlossenes Volumen der ultraschalltransparenten Flüssigkeit und die Flüssigkeit im Behältnis ist druckbeaufschlagbar.

Nach dem Festlegen der Druckmessvorrichtung am entsprechenden Körperteil kann im Behältnis der Druck gesteigert werden, wobei der Druckanstieg auf das Gewebe und somit auf die messende Vene bzw. das messende Organ übertragen wird. Die Anordnung dieser Druckmessvorrichtung ist auch in Stresssituationen sowie auch durch ungeübte Untersucher, wie durch den zu Untersuchenden selbst, einfach anwendbar.

Auf besonders vorteilhafte Weise ist bei einer Weiterbildung der Druckmessvorrichtung der Festlegeabschnitt von wenigstens einem weiteren Gehäuseteil gebildet ist, das verschwenkbar und/oder verdrehbar an dem anderen Gehäuseabschnitt angeordnet ist. Der Gehäuseabschnitt und das Gehäuseteil sind somit gegeneinander beweglich Bestandteile eines Gehäuses. Das Behältnis ist vorteilhaft an dem Gehäuseabschnitt vorgesehen.

Der Gehäuseabschnitt und der wenigstens eine weitere Gehäuseteil bilden zusammen vorteilhaft eine um den Körperteil herum anordnenbare Manschette aus.

Die Anordnung einer derartigen Druckmessvorrichtung an einem Körperteil ist besonders einfach.

Die wenigstens zwei gegeneinander beweglichen Bestandteile des Gehäuses sind beispielsweise über ein vorteilhaft scharnierähnliches Verbindungsmittel mit einander verbunden, so dass diese leicht aufklappbar und zusammenklappbar bzw. gegeneinander verschwenkbar sind. Dabei kann das Verbindungsmittel derart ausgebildet sein, dass die Bestandteile des Gehäuses seitlich zueinander oder aufeinander zu bzw. voneinander weg verschwenkbar sind. An den freien Enden sind weiter vorteilhaft Teile einer Verriegelungseinrichtung vorgesehen, welche im zusammengeführten Zustand der Bestandteile des Gehäuses miteinander verriegeln bzw. verriegelbar sind, was eine sichere Anordnung der Druckmessvorrichtung an einem Körperteil ermöglicht. Die Verriegelungseinrichtung ist vorteilhaft derart ausgebildet, dass diese einfach verriegelbar jedoch auch, vorteilhaft ohne grossen Kraftaufwand, lösbar ist. Diese Massnahme wirkt sich vorteilhaft auf die Handhabbarkeit der Druckmessvorrichtung aus.

Die Manschette ist vorteilhaft derart ausgebildet, dass diese im geschlossenen Zustand den Körperteil vollständig umgibt, um welches die Manschette angeordnet wurde. Die Manschette wird beispielsweise an einem Arm, an einem Bein, am Hals oder am Rumpf angeordnet und wird entsprechend des Orts der Anwendung entsprechend in deren Abmessungen bzw. Ausgestaltungen ausgebildet. Je nach Körperteil, an welchem die Manschette angeordnet wird, kann dieses auch mehr als zwei Gehäusebestandteile aufweisen. Ebenso können die Gehäusebestandteile asymmetrisch konfiguriert sein, um bspw. besonderen Gegebenheiten für die Ultraschallmessung oder den Druckverhältnissen im Behältnis Rechnung zu tragen.

In einer alternativen Ausführungsform kann an den freien Enden auch eine Spannvorrichtung, z. B. in Form zumindest eines Spannbandes, vorgesehen sein, mit welcher die Gehäuseteile an dem entsprechenden Körperteil fixierbar sind.

Auf besonders vorteilhafte Weise ist bei einer Weiterbildung der Druckmessvorrichtung zumindest ein Fixierungsbehältnis an dem wenigstens einen Gehäuseteil und/oder benachbart zu dem Behältnis mit der ultraschalltransparenten Messflüssigkeit vorgesehen, welches eine sichere Positionierung der Druckmessvorrichtung an dem entsprechenden Körperteil ermöglicht. Dieses zumindest eine Fixierungsbehältnis ist vorteilhaft elastisch ausgebildet und besonders vorteilhaft mit einem gasförmigen oder flüssigen Fluid befüllt.

Auf besonders vorteilhafte Weise ist bei einer Weiterbildung der Druckmessvorrichtung in dem Gehäuseabschnitt eine Aufnahme zum zumindest bereichsweisen Aufnehmen des Behältnisses vorgesehen, so dass das Behältnis in Bezug auf die Ultraschallmesseinheit immer optimal ausgerichtet ist.

Auf besonders vorteilhafte Weise ist bei einer Weiterbildung der Druckmessvorrichtung ein Drucksensor zur Messung des Druckes in der Flüssigkeit vorgesehen. Auf diese Weise ist bzw. wird vorteilhaft vermieden, dass die Lage einer Messapparatur - wie im Stand der Technik - nach dem Prinzip der kommunizierenden Röhren den Druck in dem Behältnis (Druckmesskapsel) beeinflusst. Auf besonders vorteilhafte Weise kann in diesem Zusammenhang daher auf dem Messvorgang vorangehende Kalibrierungsschritte (Nulleichung) verzichtet werden.

Der Drucksensor ist vorteilhaft im Gehäuseabschnitt bzw. in einem der Gehäusebestandteile angeordnet und besonders vorteilhaft direkt in diesem integriert. Auf diese Weise ist die Druckmessvorrichtung leicht transportabel und kann eigenhändig von einer Person bedient werden.

Auf besonders vorteilhafte Weise ist bei einer Weiterbildung der Druckmessvorrichtung eine Druckerzeugungseinrichtung zur Druckbeaufschlagung zumindest der ultraschalltransparenten Flüssigkeit in dem Behältnis vorgesehen, so dass ein entsprechender Druck im Behältnis erzeugbar ist. Ist die Auflageseite des Behältnisses flexibel ausgebildet, kann sich diese bei zunehmendem Druck im Behältnis an die Oberfläche des Gewebes anpassen, wodurch eine zuverlässige Messung ermöglicht wird. Vorteilhaft wird mit der Druckerzeugungseinrichtung ein bestimmter Referenzdruck in dem Behältnis erzeugt, welcher als Ausgangswert für die nachfolgenden Messungen bzw. Auswertungen dient.

Die Druckerzeugungseinrichtung ist vorteilhaft im Gehäuseabschnitt bzw. in zumindest einem der Gehäusebestandteile angeordnet und besonders vorteilhaft direkt in diesem integriert. Auf diese Weise ist die Druckmessvorrichtung leicht transportabel und kann eigenhändig von einer Person bedient werden.

Weist die Druckmessvorrichtung zumindest ein Fixierungsbehältnis auf, kann die Druckerzeugungseinrichtung zur Beaufschlagung der ultraschalltransparenten Messflüssigkeit gegebenenfalls auch zur Druckbeaufschlagung eines Fluids in dem zumindest einen Fixierungsbehältnis ausgestaltet sein. Alternativ wird zur Druckbeaufschlagung eines Fluids in dem zumindest einen Fixierungsbehältnis eine separate Druckerzeugungseinrichtung vorgesehen, welche vorteilhaft in dem entsprechenden Gehäusebestandteil angeordnet ist, an bzw. in welchem das zumindest eine Fixierungsbehältnis vorgesehen ist.

Auf besonders vorteilhafte Weise ist bei einer Weiterbildung der Druckmessvorrichtung eine Ansteuerungseinheit zur Ansteuerung einer zu messenden Vene bzw. eines zu messenden Organs vorgesehen. Die Ansteuerungseinheit ist vorteilhaft derart ausgebildet, dass die effektive Position der zu messenden Vene bzw. des zu messenden Organs in Bezug auf die Ultraschallmesseinheit bestimmt wird. Zu diesem Zweck kann die Ansteuereinheit einen Ultraschallwandler beinhalten, der Ultraschallwellen aussendet und deren Echos wieder einfängt. Aus der Differenz dieser Signale erstellt der Ultraschallwandler dann das eigentliche Ultraschallbild des unterliegenden Gewebes inklusive der zu messenden Vene bzw. des zu messenden Organs.

Beispielsweise wird dem Untersucher durch die Ansteuerungseinheit oder einer damit verbundenen, entsprechend ausgebildeten Anzeigeeinheit angezeigt bzw. mitgeteilt, dass eine Justierung der Druckmessvorrichtung erforderlich ist bzw. dass die korrekte Position für eine einwandfreie Messung erreicht ist. Alternativ umfasst die Ansteuerungseinheit eine Justiereinrichtung bzw. ist mit einer solchen steuermässig verbunden, mittels welcher die Ultraschallmesseinheit oder zumindest der Ultraschallkopf relativ in eine entsprechende Messposition positionierbar ist.

Besonders vorteilhaft umfasst eine Ansteuerungseinheit einen Ultraschallmesskopf, der vorteilhaft mit einer Wandelelektronik und besonders vorteilhaft mit einer Auswerteelektronik bestückt ist.

Die Ansteuerungseinheit ist vorteilhaft im Gehäuseabschnitt bzw. in zumindest einem der Gehäusebestandteile angeordnet und besonders vorteilhaft direkt in diesem integriert. Auf diese Weise ist die Druckmessvorrichtung leicht transportabel und kann eigenhändig von einer Person bedient werden.

Besonders vorteilhaft können Ausgangsmesswerte des Drucksensors bei Weiterbildungen der erfindungsgemässen Druckmessvorrichtungen über ein Verbindungskabel oder Funk weitergegeben werden. Dadurch ist die erfindungsgemässe Druckmessvorrichtung flexibler einsetzbar, bzw. eine nachgeordnete Auswerteeinrichtung kann weniger komplex ausgeführt werden, da eine Vorverarbeitung der Signale bereits im Bereich des Drucksensors stattfinden kann.

Auf besonders vorteilhafte Weise ist bei einer Weiterbildung der Druckmessvorrichtung eine Weiterverarbeitungseinheit zur Verarbeitung der vom Drucksensor ermittelten Werte vorgesehen. Auf diese Weise können komplexere Messwerte, insbesondere Messreihen, in einen Zusammenhang gebracht werden und systematische Messfehler eliminiert werden.

Die Weiterverarbeitungseinheit ist vorteilhaft im Gehäuseabschnitt bzw. in zumindest einem der Gehäusebestandteile angeordnet und besonders vorteilhaft direkt in diesem integriert. Auf diese Weise ist die Druckmessvorrichtung leicht transportabel und kann eigenhändig von einer Person bedient werden.

Auf besonders vorteilhafte Weise ist bei einer Weiterbildung der erfindungsgemässen Druckmessvorrichtung eine Anzeigeeinheit vorgesehen, um die ermittelten bzw. weiterverarbeiteten Messwerte übersichtlich darzustellen. Mittels dieser Anzeigeeinheit können vorteilhaft auch die eigentlichen Ultraschallbilder und weiter vorteilhaft auch Daten der Bildauswertung (z. B. Qualitätsdaten, Statusdaten, Statistikdaten, Alarmwerte, etc.) dargestellt werden.

Besonders vorteilhaft kann, insbesondere sofern die Weiterverarbeitungseinrichtung im Bereich des Drucksensors vorgesehen ist und in der Lage ist, komplexe Verarbeitungsaufgaben zu erledigen, die Weiterverarbeitungseinheit im Wesentlichen lediglich aus der Anzeigeeinheit bestehen.

Vorteilhafterweise kann je nach Ausführungsform die Anzeigeeinheit an der Druckmessvorrichtung oder in einer separaten Gehäuseeinheit angeordnet sein. Dies erlaubt eine grösstmögliche Flexibilität bei der Ausgestaltung der erfindungsgemässen Druckmessvorrichtung und eine genaue Anpassung an den jeweiligen Anwendungsfall.

Besonders vorteilhaft kann bei einer Weiterbildung der erfindungsgemässen Druckmessvorrichtung mittels Funktionstasten die Messung ferngesteuert eingeleitet werden, bzw. können mittels Funktionstasten spezielle Auswertungsalgorithmen oder Darstellungsarten auf der Anzeigeeinheit eingestellt werden. Insbesondere kann beispielsweise mittels der Funktionstasten ein automatischer Messvorgang eingeleitet werden, bzw. eine manuelle Druckmessung mit Unterstützung der Anzeige durchgeführt werden.

Besonders vorteilhaft ist bei einer Weiterbildung der erfindungsgemässen Druckmessvorrichtung eine Halterungseinrichtung für die Ultraschallmesseinheit bzw. zumindest für einen Ultraschallmesskopf an oder im Gehäuseabschnitt vorgesehen. Auf diese Weise kann schnell und zuverlässig eine Messeinheit aus Ultraschallmesskopf und Druckmessvorrichtung gebildet werden.

In einer alternativen Ausführungsform der Erfindung ist die Ultraschallmesseinheit bzw. zumindest der Ultraschallmesskopf integrierter Bestandteil der Druckmessvorrichtung, womit die Druckmessvorrichtung leicht transportabel ist und kompakt ausgebildet werden kann.

Bei einem erfindungsgemässen Druckmesssystem zur Druckmessung einer Vene oder eines Organs gemäss Anspruch 12 wird eine Druckmessvorrichtung gemäss einem der Ansprüche 1 bis 11 mit einer Ultraschallmesseinheit kombiniert und so zu einer Messeinheit gekoppelt oder mit dieser verbunden. Vorteilhaft werden die Druckmessvorrichtung und die Ultraschallmesseinheit mechanisch miteinander gekoppelt. Alternativ zu einer formschlüssigen Kopplung kann auch eine kraftschlüssige Kopplung vorgesehen werden.

Bei einem erfindungsgemässen Verfahren zur Druckmessung einer Vene oder eines Organs mit einem Druckmesssystem nach Anspruch 12 wird dieses um den entsprechenden Körperteil, unter dem sich eine zu messende Vene bzw. das zu messende Organ befindet, herumgelegt sowie vorteilhaft an diesem fixiert. Dann wird solange der Druck im Behältnis und somit auf das Gewebe gesteigert, bis sich aus der Ultraschallmessung ergibt, dass die Vene kollabiert bzw. das Organ einen bestimmten Verformungsgrad erreicht hat. Das von der Ultraschallmesseinheit abgegebene Signal wird dazu verwendet, den Zeitpunkt einer Druckmessung festzulegen, wobei der zu diesem Zeitpunkt anliegende Druck von der Druckmessvorrichtung als Venendruck bzw. Organdruck bestimmt wird.

In vorteilhafter Weise können je nach Komplexität der Druckmessvorrichtung bereits vorverarbeitete Signale an eine Anzeigeeinheit und/oder Auswerteeinheit per Funk oder Verbindungskabel weitergeleitet werden. Auf diese Weise lässt sich je nach Anwendung vorhandener Infrastruktur das Verfahren zur Venen- bzw. Organdruckmessung optimal ausbilden. Ist ein Drucksensor vorgesehen, erfolgt die Auswertung von Messinformation vorzugsweise zumindest teilweise im Bereich des Drucksensors und diese ausgewerteten Messinformationen werden dann über Funk oder Verbindungskabel an eine Anzeigeeinheit und/oder Auswerteeinheit weitergeleitet.

Als Zeitpunkt der Druckmessung wird der Zeitpunkt des Kollabierens einer Vene oder das Erreichen eines bestimmten Verformungsgrades eines Organs festgelegt.

Ein Organ muss für die Druckmessung nicht vollständig im Ultraschall abgebildet werden, da die Druckmessung mit Gewährleistung von wiederholbaren und zuverlässigen Messergebnissen auch nur an einem Organteil erfolgen kann. Dadurch sind auch grosse Organe, wie z. B. eine Leber, messbar.

Besonders vorteilhaft lässt sich bei einer Weiterbildung des erfindungsgemässen Verfahrens der Zeitpunkt der Druckmessung automatisch in einer Verarbeitungseinheit, beispielsweise durch Computerauswertung der Ultraschallmesswerte, in Verbindung mit den Druckmesswerten bestimmen.

Auf besonders vorteilhafter Weise wird bei einem Verfahren zur Druckmessung einer Vene oder eines Organs der Zeitpunkt des Kollabierens einer Vene oder das Erreichen eines bestimmten Verformungsgrades eines Organs über ein bildverarbeitendes und/oder bildauswertende bzw. bildgebendes Verfahren bestimmt, welche Ultraschallbilder analysieren.

Vorzugsweise wird eine Software zur Durchführung des bildverarbeitenden Verfahrens zur Verfügung gestellt. Eine solche Software stellt für sich eine eigenständige Erfindung dar. Der kollabierte Zustand einer Vene wird dabei durch die Umrechnung von Rohdaten (Pixeln) in eine durchströmte Querschnittsfläche der Vene erkannt. Als Kriterium für das Kollabieren kann eine gegenüber dem Querschnitt der unbelasteten Vene beliebige Querschnittsreduktion definiert werden. Bei Organen wird demensprechend durch die Verschiebung von gewebebedingten Pixelanormalitäten (z. B. Blutgefässe, Dichteunterschiede, etc.) der Verformungsgrad des Organs oder des Organteils bestimmt. Wie bereits ausgeführt wurde, muss für eine Druckmessung mit der erfindungsgemässen Druckmessvorrichtung eines Organs dieses nicht vollständig im Ultraschall abgebildet werden.

In einer vorteilhaften Weiterbildung der Software ist das automatische Erkennen des Beginns des Messzyklus durch den Beginn des Deformierens der Vene bzw. durch den Beginn der Verringerung der durchströmten Querschnittsfläche definiert. Bei Organen oder Organteilen wird vorteilhaft der Zeitpunkt gewählt, bei dem sich eine zuvor festgestellte Pixelanormalität zu verschieben beginnt.

In einer weiteren vorteilhaften Weiterbildung der Software ist das automatische Erkennen der Messqualität durch Bestimmen der Lage der beobachteten Vene bzw. des Organs oder Organteils gegenüber der Messachse des Ultraschallmesskopfs bzw. des Ultraschallwandlers und/oder des Behältnisses mit der Flüssigkeit der Druckmessvorrichtung definiert. Vorteilhaft wird dabei auch die Lage der Vene bzw. des Organs gegenüber benachbarten und insbesondere darunterliegenden Strukturen berücksichtigt, wie Knochen oder dergleichen.

In einer vorteilhaften Weiterbildung der Software erfolgt ein automatischer Entscheid über akzeptable oder nicht akzeptable Messung durch Berücksichtigung und Analyse aller vorgenannten Daten.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind. Dabei können die in den Ansprüchen und in der Beschreibung erwähnten Merkmale jeweils einzeln für sich oder in beliebiger Kombination erfindungswesentlich sein.

Die Bezugszeichenliste und die Ansprüche sind Bestandteil der Offenbarung. Die Figuren werden zusammenhängend und übergreifend beschrieben. Gleiche Bezugszeichen bedeuten gleiche Bauteile.

Es zeigen dabei:
- Fig. 1: Ein erstes Ausführungsbeispiel einer erfindungsgemässen, als Manschette ausgebildeten Druckmessvorrichtung, welche hier beispielhaft als Armmanschette ausgeführt und über ein Verbindungskabel mit einer separaten Gehäuseeinheit verbunden ist, in einer Schnittdarstellung;
- Fig. 2: die Druckmessvorrichtung gem. Figur 1 mit der separaten Gehäuseeinheit;
- Fig. 3: eine zweite Ausführungsform einer als Manschette ausgeführten, erfindungsgemässen Druckmessvorrichtung;
- Fig. 4: die Druckmessvorrichtung gem. Figur 3 in einer Schnittdarstellung;
- Fig. 5: eine dritte Ausführungsform einer als Manschette ausgeführten, erfindungsgemässen Druckmessvorrichtung, die über Funk mit einer separaten Gehäuseeinheit gekoppelt ist; und
- Fig. 6: eine vierte Ausführungsform einer erfindungsgemässen Druckmessvorrichtung in einer Schnittdarstellung.

Die Figuren 1 und 2 zeigen eine erfindungsgemässe Druckmessvorrichtung 100, welche als Manschette - in diesem Fall als Armbandmanschette - ausgebildet ist. Bei dieser Ausführungsform der Druckmessvorrichtung 100 handelt es sich bevorzugt um ein vollautomatisches Venendruck-Messgerät.

Die hier gezeigte Druckmessvorrichtung 100 weist ein zweiteiliges Gehäuse 105 auf, mit einem ersten Gehäuseabschnitt 115 und einem zweiten Gehäuseteil 120, welche über ein Scharnier 125 miteinander und zueinander schwenkbar verbunden sind. An ihren dem Scharnier 125 gegenüberliegenden Enden ist jeweils ein Teil einer Verriegelungseinrichtung 135 vorgesehen. Im in der Figur 1 dargestellten geschlossenen Zustand der Druckmessvorrichtung 100 verriegeln die Teile der Verriegelungseinrichtung 135 miteinander, so dass die Druckmessvorrichtung 100 an der Extremität, hier an dem Unterarm 10, fixiert ist.

Die beweglichen Bestandteile der als Manschette ausgebildeten Druckmessvorrichtung 100 können neben Scharnieren alternativ oder ergänzend dazu auch über Spannvorrichtungen und/oder über ein Klettband aneinander befestigt sein (hier nicht dargestellt).

An der Innenseite des ersten Gehäuseabschnitts 115 ist das Behältnis 170 für die ultraschalltransparente Flüssigkeit 11 vorgesehen, welches vorteilhaft formschlüssig an dem ersten Gehäuseabschnitt 115 in einer Aufnahme 116 festgelegt ist. Alternativ ist das Behältnis 170 in dem ersten Gehäuseabschnitt 115 lediglich eingelegt.

Vorteilhaft ist das Behältnis 170 lösbar in bzw. an dem Gehäuseabschnitt 115 festgelegt. Diese Lösung bietet den Vorteil Behältnisse mit unterschiedlichen Abmessungen dem Untersucher bereitzustellen, welche abhängig von den Ausdehnungen der Extremität von dem Untersucher in dem Gehäuse 105 der Druckmessvorrichtung 100 vorgesehen werden. Somit muss dem Untersucher für die Anwendung der Druckmessvorrichtung 100 an Körperteilen mit unterschiedlichen Abmessungen nur eine Dimension des Gehäuses 105 zur Verfügung gestellt werden.

Der dem ersten Gehäuseabschnitt 115 abgewandte Abschnitt des Behältnisses 170 bildet eine Auflageseite 171 zur Auflage auf die Gewebeoberfläche 15 des Unterarms 10 und der dem ersten Gehäuseabschnitt 115 zugewandte Abschnitt des Behältnisses 170 bildet eine Anlageseite 172 für die Anlage der Ultraschallmesseinheit 190 an dem Behältnis 170. Durch die Anlageseite 172 und die Auflageseite 171 tritt ein von der Ultraschallmesseinheit 190 ausgesendeter Ultraschall durch das Behältnis 170 hindurch in den Unterarm 10 ein und vom Gewebe 14 reflektierter Ultraschall gelangt wieder zurück zur Ultraschallmesseinheit 190. Die Auflageseite 171 ist flexibel ausgebildet, damit diese nahezu vollständig auf der Gewebeoberfläche 15 des Unterarms 10 aufliegen bzw. sich an diese anpassen kann.

In praktischen Versuchen hat sich gezeigt, dass das Material und die Auslegung dieser Auflageseite 171 des Behältnisses einen grossen Einfluss auf die Qualität der Ultraschallmessung haben. In zahlreichen Versuchen wurde Silikonelastomer als geeignet ermittelt. Ein bevorzugter Materialtyp ist MVQ (internationales Kurzzeichen), das sich durch folgende Daten auszeichnet: Härte 50° Shore A; Dichte 1,15 g/cm³; Aussehen milchig-transparent. Die Dicke der Auflagemembran sollte möglichst gering sein, zum Beispiel 0,4 - 0,5 mm. Das Material MVQ konnte problemlos mit einer Dicke von 1 mm eingesetzt werden. Kleinere Dicken sind von Vorteil, haben jedoch den Nachteil, dass sie sich leichter beschädigen lassen und schneller abnutzen. Dickere Membranen in der Grössenordnung von 3 mm und mehr beeinträchtigen dagegen das Ultraschallmessergebnis beträchtlich. Zwischen der Auflageseite 171 des Behältnisses 170 und der Gewebeoberfläche 15 wird vorzugsweise ein ultraschalltransparentes Gel 17 eingebracht, um die Ankopplung des Ultraschalls in das Gewebe 14 zu verbessern. Die Anlageseite 172 des Behältnisses 170 weist bevorzugt eine geringe Biegsamkeit als die Auflageseite 171 auf.

Als Material für die Anlageseite 172 des Behältnisses 170 hat sich insbesondere Teflon® (Polyfluorethylen, Dupont) in einer Dicke von ca. 0.05 - 0,7 mm als geeignet erwiesen. Dicken in der Grössenordnung von 1 mm und mehr führen zu einer deutlichen Verschlechterung der Qualität der Ultraschallmessung. Ab einer Dicke von 3 mm der Anlageseite 172 waren die Messungen praktisch unbrauchbar.

In einer weiteren Ausführungsform des Behältnisses 170 wird als Material vorzugsweise Polytetrafluoräthylen verwendet, abgekürzt PFTE und als Markenname mit der Bezeichnung Teflon von Dupont registriert. Der Vorteil dieses Materials beim Einsatz in Ausführungsbeispielen der Erfindung liegt darin, dass weniger Verluste auftreten, keine Vorspannung auftritt und damit eine genauere Druckmessung möglich ist. Die Auflageseite 171 des Behältnisses 170 kann beispielsweise aus Schrumpfteflon ausgeführt sein. Falls die Anlageseite 172 des Behältnisses 170 eine geringe Elastizität aufweist, hat dies den Vorteil, dass weniger Verluste auftreten und die Messung dadurch genauer wird.

Grösse und Form des Behältnisses 170 können je nach Anwendung und Aufgabe variiert werden. Beispielsweise können anstelle eines blasen- bzw. schlauchförmigen Behältnisses 170 die Anlageseite 172 und die Auflageseite 171 auch in einem Rahmen z. B. aus Metall gehalten werden. Im Hinblick auf einen Rahmen, der keine besonderen Eigenschaften hinsichtlich Ultraschalls aufweisen muss, kann also auch an andere Materialien, wie zum Beispiel Metall oder harte Kunststoffe, gegebenenfalls mit Einlagen, gedacht werden.

Es ist besonders wichtig, dass die Flüssigkeit 11 den Ultraschalldurchtritt nicht behindert und idealerweise vollständig für Ultraschall transparent ist. In diesem Zusammenhang haben sich insbesondere Glycerin-Wasser-Mischungen bewährt.

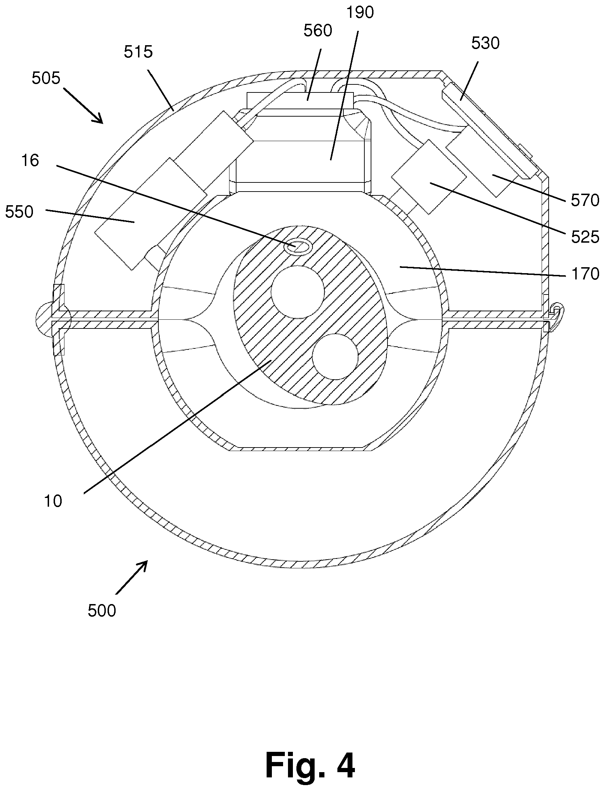

An der Innenseite des zweiten Gehäuseteils 120 ist ein Fixierungsbehältnis 180 vorgesehen, in welchem beispielsweise ein gasförmiges oder flüssiges Fluid eingeschlossen ist. Alternativ ist das Fixierungsbehältnis 180 aus einem vorteilhaft elastisch verformbaren Material gefertigt. In diesem Ausführungsbeispiel erstreckt sich das Fixierungsbehältnis 180 im Wesentlichen über den gesamten Innenkreisabschnitt des zweiten Gehäuseteils 120. Das Behältnis 170 für die Flüssigkeit 11 dagegen erstreckt sich lediglich über einen Teilbereich des Innenkreisabschnitts des Gehäuseabschnitts 115, nämlich über den entsprechenden Abschnitt der Ultraschallmesseinheit 190. Wie in den Figuren 4 und 5 ersichtlich, kann auch das Behältnis 170 sich über den gesamten Innenkreisabschnitt des Gehäuseabschnitts mit der Ultraschallmesseinheit 190 erstecken.

In dem Gehäuseabschnitt 115 ist weiter eine Ultraschallmesseinheit 190 integriert, so dass beide zusammen ein Druckmesssystem ausbilden.

Neben der fixen Kopplung oder konstruktiven Integration an oder in der Druckmessvorrichtung 100 sind verschiedene Ankopplungen an bekannte Ultraschallköpfe denkbar. Zur Gewährleistung einer zuverlässigen Messung ist in einem solchen Fall vorzugsweise eine Halterungseinrichtung 200 vorgesehen, welche die Ultraschallmesseinheit 190 nach deren Anordnung im Gehäuse 105 der Druckmessvorrichtung 100 in der richtigen Ausrichtung zum Behältnis 170 in dem Gehäuse 105 fixiert.

Die Halterungseinrichtung 200 umfasst zum Beispiel Federelemente, um eine Ultraschallmesseinheit 190 oder einen Ultraschallmesskopf im Gehäuse 105 zu halten. In einer Variante oder ergänzend dazu sind Klettverschlüssen vorgesehen, welche eine stabile, druckfreie Fixierung der Ultraschallmesseinheit 190 auf dem Behältnis 170 gestatten. Dazu sind beispielsweise schwenkbare Flügel vorgesehen, welche um Stäbe schwenkbar sind und zur Fixierung der Ultraschallmesseinheit 190 mit Klettband versehen sind. Für die Ausführung der Halterungseinrichtung 200 zur Halterung der Ultraschallmesseinheit 190 im Gehäuse 105 sind technisch beliebige Varianten denkbar. Jedoch stellt im Hinblick auf die Zuverlässigkeit der Messergebnisse und dem einfachen Handling der Druckmessvorrichtung eine untrennbare Verbindung zwischen der Ultraschallmesseinheit 190 und dem Gehäuse 105 bzw. eine integrierte Anordnung die bevorzugteste Lösung dar.

Kommt eine separate, bedarfsweise an die Druckmessvorrichtung 100 anordnenbare Ultraschallmesseinheit 190 zur Anwendung, wird vorzugsweise zwischen der Anlageseite 172 des Behältnisses 170 und der Ultraschallmesseinheit ein ultraschalltransparentes Gel vorgesehen, womit zuverlässige Messergebnisse ohne Störungen aufgrund dieses Übergangbereichs gewährleistet werden.

Die Ultraschallmesseinheit 190 ist über ein Verbindungskabel 195 und das Behältnis 170 ist über eine Leitung 175 zur Druckmessung jeweils mit einer separaten Gehäuseeinheit 210 über einen Steckverbinder 250 verbunden bzw. an dieser angekoppelt. Eine Druckmessung kann mittels kommerziell erhältlicher Druckwandler vorgenommen werden, indem der Druck in der im Behältnis 170 befindlichen ultraschalltransparenten Flüssigkeit 11 gemessen wird.

An der separaten Gehäuseeinheit 210 sind eine Anzeigeeinheit 130 und Funktionstasten 140, 150 und 160 sowie 230 und 240 vorgesehen. Über die Funktionstasten 140, 150, 160, 230 und 240 der separaten Gehäuseeinheit 210 kann beispielsweise ein Messvorgang gestartet und/oder eine in der separaten Gehäuseeinheit 210 vorgesehene Verarbeitungseinheit gesteuert werden. Ferner können bspw. Datenauswertungen getätigt werden. Beispielsweise können Analysen von mehreren gespeicherten Messreihen durchgeführt werden, um systematische Messfehler zu bestimmen. Über das Verbindungskabel 195 können Steuerbefehle an die Druckmessvorrichtung 100 gegeben werden, um das Auftreten systematischer Fehler zu verringern.

Auf der Anzeigeeinheit 130 können bspw. Druckmesswerte angezeigt werden. Ferner besteht die Möglichkeit, parallel dazu auch ein Abbild der Vene 16 darzustellen und diesem Abbild die aktuell im Gewebe 14 gemessenen Druckwerte gegenüberzustellen.

Die Druckmessvorrichtung 100 beinhaltet aufgrund des Gehäuses 105 mit zwei Gehäusebestandteilen 115 und 120 mindestens eine Trennebene, mittels derer die Druckmessvorrichtung 100 so geöffnet werden kann, dass der Unterarm 10 einlegbar ist. Nach dem Verschliessen der Druckmessvorrichtung 100 wird bspw. die Messflüssigkeit 11 (vorteilhaft Glycerin oder eine ähnliche Flüssigkeit) automatisch in das Behältnis 170 auf der Innenseite des ersten Gehäuseabschnitts 115 z. B. aus dem separaten Gehäuseeinheit 210 gefördert. Der Druck in der Flüssigkeit 11 wird dabei bevorzugt periodisch oder kontinuierlich gemessen. Das Behältnis 170, welches bspw. schlauchförmig ausgebildet sein kann, wird dabei aufgebläht, wobei sich die Auflagefläche 171 des Behältnisses 170 an die unterliegende Hautoberfläche des eingelegten Unterarms 10 anlegt. Somit wird auch der Druck in der ultraschalltransparenten Flüssigkeit 11 auf die Gewebeoberfläche 15 des Unterarms 10 übertragen. Von dieser wird der Druck weiter in das innenliegende Gewebe 14 des Unterarms 10 geleitet, was zum Kollabieren der im Gewebe 14 eingelagerten, zu messenden Vene 16 führt. Gleichzeitig wird mittels der in der Druckmessvorrichtung 100 eingebauten Ultraschallmesseinheit 190 die entsprechende Venenöffnung gemessen. Durch Vergleich der gemessenen Venenöffnung mit dem gemessenen Druck kann der venöse Blutdruck bestimmt werden.

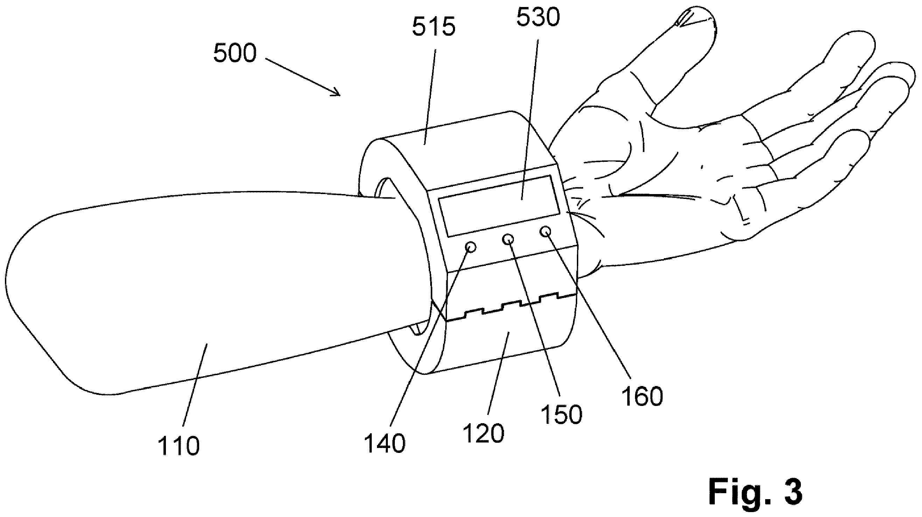

Die in den Figuren 3 und 4 dargestellte Druckmessvorrichtung 500 unterscheidet sich von der zuvor beschriebenen Druckmessvorrichtung 100 hauptsächlich dadurch, dass die Bestandteile des Druckmesssystems in dem Gehäuse 505 integriert sind.

Neben der Ultraschallmesseinheit 190 sind des Weiteren in dem ersten Gehäuseabschnitt 515 eine Druckerzeugungseinrichtung 550 vorgesehen, mittels derer der Druck der ultraschalltransparenten Flüssigkeit 11 im Behältnis 170 gesteigert werden kann. Beispielsweise wird der Druck durch einen mechanischen Kolben der Druckerzeugungseinrichtung 550 erhöht, der das geschlossene Volumen komprimiert. Die Druckerzeugungseinrichtung 550 ist bspw. elektrisch, pneumatisch und/oder hydraulisch betrieben und wird entsprechend des verwendeten Mediums ausgebildet. Weiter ist in dem ersten Gehäuseteil 515 ein Drucksensor 525 vorgesehen, der den Druck in der Flüssigkeit 11 bevorzugt periodisch oder kontinuierlich misst.

Daneben ist eine Ansteuerungseinheit 560 ebenfalls in dem ersten Gehäuseteil 515 integriert. Die Ansteuerungseinheit 560 ermöglicht eine Identifizierung der zu messenden Vene 6 in Bezug auf die Position des Ultraschallkopfs der Ultraschallmesseinheit 190, so dass die Druckmessvorrichtung 500 gegebenenfalls korrekt ausgerichtet werden kann und somit präzise und wiederholbare Messergebnisse mit der Druckmessvorrichtung 500 erreicht werden. Des Weiteren ermöglicht die Ansteuerungseinheit 560 vorteilhaft auch ein Bestimmen des Kollabierungsdruck bei einer zu messenden Vene 16 (z. B. Vene 16 kollabiert bei Druck XY) bzw. Bestimmen des Verformungsgrades bei einem zu messenden Organ (z. B. dessen bestimmter Verformungsgrad wird bei Druck YX erreicht). "XY" und "YX" sind in diesem Zusammenhang Beispiele für fiktive Werte zur Verdeutlichung der Erfindung.

Bezüglich der vorteilhaften Materialeigenschaften und bevorzugten Materialarten für das Behältnis 170 wird auf die vorgenannten Ausführungen zu dem bei der Ausführungsform gem. den Figuren 1 und 2 beschriebenen Behältnis 170 verwiesen, da diese vorteilhaft identisch oder zumindest gleichartig sind.

Die vorgenannten im ersten Gehäuseabschnitt 515 angeordneten Bestandteile sind direkt oder indirekt mit einer ebenfalls in dem ersten Gehäuseabschnitt 515 vorgesehenen Recheneinheit 570 als Verarbeitungseinheit verbunden, in welcher die ermittelten Werte ausgewertet und z. B. für eine Anzeige aufbereitet werden. Diese Verarbeitungseinheit ist beispielsweise als Prozessor in Form eines Mikroprozessors oder Mikrocontrollers ausgeführt. In diesem Ausführungsbeispiel werden die aufbereiteten Daten auf einer an dem ersten Gehäuseabschnitt 515 angeordneten Anzeigeeinheit 530 dargestellt.

An dem ersten Gehäuseabschnitt 515 sind zudem Funktionstasten 140, 150 und 160 vorgesehen, deren Funktion der im Zusammenhang mit der Ausführungsform gemäss den Figuren 1 und 2 beschrieben Funktion entsprechen kann. Eine dieser Funktionstasten 140, 150 und 160 kann beispielsweise zum manuellen Starten der Druckmessung mit der Druckmessvorrichtung 500 dienen.

In diesem Zusammenhang ist es wichtig zu verstehen, dass beim Stand der Technik die Druckmessung nicht in einem geschlossenen Gehäuse erfolgt. Dies hat zur Folge, dass nach dem Prinzip der kommunizierenden Röhren die Lage einer Druckmessvorrichtung oder einer Druckregelungseinrichtung, wie sie in US 6,086,533 A beschrieben ist, bei einer ungenügenden vorgängigen Nulleichung zu Verfälschungen des Messergebnisses führen kann. Ferner ist zu festzustellen, dass die angekoppelten Flüssigkeit führenden Bauelemente, wie zum Beispiel die Leitung 175 zum Druckmessgerät und dort befindliche weitere Flüssigkeit führende Bauelemente, durch ihre elastischen Eigenschaften und den Sachverhalt, dass der Flüssigkeit eine grössere Fläche zur Verfügung steht, das Messergebnis erheblich beeinträchtigen können. Insbesondere ist in diesem Zusammenhang zu berücksichtigen, dass das Flüssigkeitsvolumen in dem Behältnis für einen verbesserten Ultraschall Durchlass relativ gering ausfällt und ein entsprechendes grösseres Flüssigkeitsvolumen, welches durch die Leitung zum Druckmessgerät und die flüssigkeitsführenden Bauteile im Druckmessgerät hinzukommen, eine gewisse Messträgheit bedingen und daher die Messung verfälschen können. Insbesondere entfällt bei einer derartigen erfindungsgemässen Vorrichtung ein Kalibrierungsvorgang (Nulleichung), um den Einfluss von unterschiedlichen Flüssigkeitsniveaus zwischen Behältnis (Druckmesskapsel) und Druckmessvorrichtung zu kompensieren.

Die Druckmessvorrichtung 500 ist vorteilhaft als autonome Einheit mit einer internen Stromversorgung, z. B. mit einer Batterie, zur Versorgung der im Gehäuse 505 angeordneten Bauteile ausgebildet.

In der Figur 5 ist eine teilautonome Ausführungsform einer Druckmessvorrichtung 600 gezeigt, bei der eine drahtlose Übertragung von Daten von der Druckmessvorrichtung 600 auf eine separate Gehäuseeinheit 210 erfolgt. Dazu weist die Druckmessvorrichtung 600 einen Sender 615 und die separate Gehäuseeinheit 210 einen Empfänger 620 auf. Der Sender 615 und der Empfänger 620 sind vorteilhaft derart ausgebildet, dass die Datenübertragung bidirektional und somit auch von der separaten Gehäuseeinheit 210 auf die Druckmessvorrichtung 600 erfolgen kann.

Die in Figur 5 dargestellte separate Gehäuseeinheit 210 entspricht vorteilhaft der in der Figur 2 dargestellten Ausführung, lediglich mit einem Empfänger 620. Dadurch sind mit einem Typ von separater Gehäuseeinheit 210 beide Varianten der Druckmessvorrichtungen 100 bzw. 600 verwendbar.

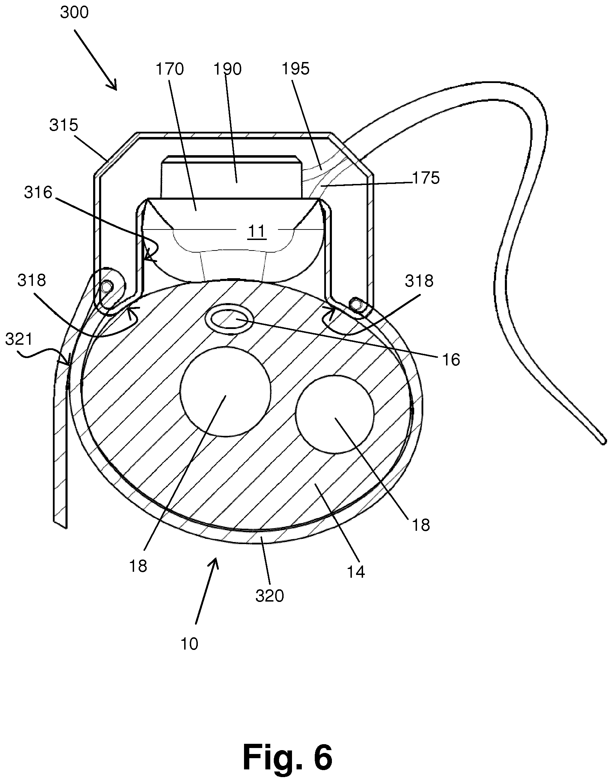

Die in der Figur 6 gezeigte Druckmessvorrichtung 300 weist ein Gehäuseabschnitt 315 mit dem Behältnis 170 und ein Festlegeabschnitt 320 zum Festlegen der Druckmessvorrichtung an dem Körperteil 10 auf. Als Festlegeabschnitt 320 kommt z. B. ein einfacher Riemen oder ein Klettverschluss 321 zur Anwendung, so dass die Druckmessvorrichtung 300 an dem entsprechenden Körperteil 10 angeordnet werden kann, ohne dass eine zweite, feste Schale - wie ein Gehäuseteil - dazu notwendig ist und ohne dass das zu Gewebe 14 durch die Festlegung bereichsweise komprimiert wird, was zu ungenauen Messergebnissen führen kann.

Das Behältnis 170 ist fast vollständig in einer Aufnahme 316 des Gehäuseabschnitts 315 angeordnet. Der Gehäuseabschnitt 315 bzw. dessen seitliche Stützabschnitte stützen sich lediglich mit ihren Kontaktbereichen 318 auf dem Körperteil 10 ab. Die Ultraschallmesseinheit 190 befindet sich dabei ausserhalb des zu messenden Bereichs, so dass durch die Abstützung keine Verfälschung der Messungen entsteht. Diese Ausführungsform zeichnet sich insbesondere durch deren Flexibilität aus. Sie kann ohne besondere konstruktive Massnahmen auf Körperteile mit sehr stark variierender Geometrie und Grösse aufgebracht werden.

Insbesondere lassen sich mit den erfindungsgemässen Druckmessvorrichtungen 100, 300, 500 bzw. 600 Venendrücke und Organdrücke bestimmen. Dies erlaubt bspw. eine schnelle und kostengünstige Messung des peripheren und zentralen Venendruckes (ZVD). Die bisherige konventionelle Messung erfolgt invasiv durch Einführen eines Katheters ins Herz. Eine solche Messung hat eine Komplikationsrate von ungefähr 20% und ist mit teilweise gravierenden Risiken für Patienten verbunden. Zudem dauert die Einlage des Katheters ca. 23 Minuten und muss von zwei Personen, von denen zwingend eine ein Arzt muss, vorgenommen werden. Der Eingriff ist relativ kostspielig. Demgegenüber stellt das erfindungsgemässe Druckmesssystem 100, 300, 500 bzw. 600 eine nicht-invasive schnellere, einfachere, günstigere und komplikationsfreie Messmethode zur Venendruckmessung zur Verfügung. Die Druckmessung kann hierbei durch geschultes Personal bzw. angelernte Untersucher durchgeführt werden.

Der Einsatz des erfindungsgemässen Druckmesssystems 100, 300, 500 bzw. 600 führt zur schnelleren Betriebsabläufen und erlaubt eine weitestgehend automatisierte Messung des zentralen Venendruckes, ähnlich dem von im Handel üblichen Blutdruckmessgeräten für den arteriellen Blutdruck.

Selbstverständlich ist die Messmethode nicht auf Venen beschränkt, sondern kann auch bei anderen Gewebestrukturen, insbesondere bei Organen (z. B. bei der Leber oder der Milz) und andere Körperflüssigkeiten Anwendung finden.

Vorteilhaft kann der Messvorgang automatisch gestartet werden. Sobald ein Druckimpuls vom Drucksensor 525 gemessen wird, kann zum Beispiel die Anzeigevorrichtung 130 eingeschaltet werden, ferner kann ebenso über ein geeignetes Signal die Ultraschallmesseinheit 190 gestartet werden. Ein automatischer Startvorgang hat den Vorteil, dass das erfindungsgemässe Druckmesssystem energieeffizient eingesetzt werden kann. Es ist ferner denkbar, dass über geeignete Analysemethoden automatisch der Zeitpunkt festgestellt werden kann, an dem eine Vene 16 oder ein anderes Körperflüssigkeitsgefäss kollabiert. Beispielsweise ist es denkbar, dass über ein Körperschallmikrofon die Veränderung eines Blutströmungsgeräusches die Veränderung im Strömungsgeräusch als Indikator für das Kollabieren einer Vene 16 verwendet wird. Ebenfalls ist es denkbar, über bildverarbeitende bzw. bildgebende Verfahren erzeugte Abbilder vom Inneren des Gewebes 14 in einer Extremität (z. B. Arm 10) automatisch zu analysieren, um die Lage einer Vene 16 oder eines Organs aufzuspüren und deren Geometrieveränderung zu bestimmen. Auf diese Weise kann ebenfalls eine Detektion des Kollabierens der Vene erfolgen, um den Zeitpunkt der korrekten Messung des Venendruckes festzulegen. Dieser Zeitpunkt wird dann im zeitlichen Verlauf der Druckmessung als Zeitpunkt festgelegt an dem der zentrale Venendruck ermittelt wurde.

Ferner kann vorteilhaft auf einer Anzeigeeinheit 130 bzw. 530 gleichzeitig zum Druckverlauf ein Abbild der mittels Ultraschall beobachteten Vene 16 nebeneinander oder übereinander dargestellt werden. Eine derartige Ausführungsform bietet den Vorteil einer kompakten Darstellung und erlaubt es lediglich, eine Anzeigevorrichtung 130 bzw. 530 für mehrere Anzeigefunktionen bereitstellen zu müssen.

Bei weiteren Ausführungsformen ist es beispielsweise denkbar, die von der erfindungsgemässen Druckmessvorrichtung gemessenen Messwerte zu sammeln und zur Qualitätskontrolle zu verwenden, um insbesondere die richtige Position der Druckermittlung, das heisst die korrekte Lage über einer Vene 16, nachvollziehen zu können und nachvollziehen zu können, ob die richtige und eine ausreichende Kraft für die Druckmessung auf das Gewebe 14 ausgeübt wurde.

Generell haben automatisierte Verfahren in Ausführungsformen der erfindungsgemässen Druckmessvorrichtung 100, 300, 500 bzw. 600 und des erfindungsgemässen Druckmesssystems den Vorteil, dass weniger Fehler auftreten. Ferner ist es auch denkbar, das Verfahren zur Messung der Lebersteifigkeit einzusetzen. Hierzu wird die Leber mittels des zunehmenden Drucks in der Messflüssigkeit bis zu einem bestimmten Verformungsgrad deformiert, wobei das Verhältnis von Verformungsgrad zum Druck das Mass für die Lebersteifigkeit darstellt. Alternativ kann die Lebersteifigkeit auch gemessen werden indem der Druck einer Lebervene ermittelt und im Umkehrschluss wird festgestellt, dass, je höher der Druck in der Lebervene ist, desto steifer das Lebergewebe ist.

Im Folgenden werden mögliche Benutzerfehler bei der Venendruckmessung und Massnahmen dagegen beschrieben.
- Die Anordnung der Druckmessvorrichtung ist zu weit von der Achse in Bezug auf die Zielvene entfernt und deswegen wird kein Spitzendruck auf die Vene ausgeübt. Daher sollte bevorzugt das erfindungsgemässe Verfahren zur Venendruckmessung eine Detektion der Mittelachse des Druckmesssystems und einen Vergleich derselben mit der Zielvene durchführen. Bevorzugt wird die Zielvene dabei farblich auf der Anzeigeeinheit markiert. Befindet sich die Druckmessvorrichtung nicht in einer idealen Position für eine genaue Druckmessung, erfolgt vorzugsweise eine Warnung des Untersuchers mit Aufforderung zur Korrektur der Position der Druckmessvorrichtung und es erfolgt keine Freigabe der Druckmessung.
- Die Ausübung des Drucks durch die Druckmessvorrichtung auf die Vene erfolgt ungleichmässig und führt zur asymmetrischen Gewebeverschiebung zwischen der Druckmessvorrichtung, die das Gewebe 14 und die Vene 16 berührt. Daher werden erfindungsgemäss bevorzugt Oberflächen-Verschiebungs- und elastographische Verfahren angewendet werden, um solche Fälle aufzuspüren. Vorzugsweise erfolgt in einem solchen Fall eine Warnung des Untersuchers mit Aufforderung zur Korrektur mit Aufforderung zur gleichmässigen Druckaufbringung und es erfolgt keine Freigabe der Druckmessung.
- Eine Anordnung der Druckmessvorrichtung erfolgt an einen ungeeigneten Ort. Als Abhilfe ist erfindungsgemäss eine Auswertung des Kontaktbereiches für die Druckmessvorrichtung vorgesehen. Beispielsweise wird ein geeigneter Ort zur Venendruckmessung durch Suchen nach innenliegendem Knochen 18 unterhalb der Zielvene 16 gefunden. Vorzugsweise erfolgt eine Warnung des Untersuchers mit Aufforderung zur Korrektur der Platzierung der Druckmessvorrichtung und es erfolgt keine Freigabe der Druckmessung.
- Gegebenenfalls - bei den in den Figuren 3 bis 5 beschriebenen Ausführungsformen normalerweise nicht - sollte der Systemdruck in der Messflüssigkeit 11 vor der Messung zu Null gesetzt werden (Nulleichung). Eine vorzugsweise vorgesehene automatische Kalibrierungsfunktion gestattet es dem Druckmesssystem, sich selbst vor der Messung zu kalibrieren. Dies sollte erfindungsgemäss bevorzugt durch die Detektion des gegenwärtigen Drucks zusammen mit dem dargestellten Ultraschallbild geschehen. Hierzu kann bspw. eine bestimmte Druckanstiegsrate, z. B. in Bezug auf einen durch den Untersuchenden oder das Druckmesssystem definierten Referenzdruck, in Verbindung mit einem Ultraschallbild, anhand dessen die Gewebeverschiebung bspw. anhand Anwendung elastographischer Verfahren detektiert wird, verwendet werden. Es entfällt hiermit eine Kalibrierung des Druckmesssystems zur Venendruckmessung durch den Untersucher vor Beginn der Messung und ein entsprechender Kalibrierungsfehler wird in vorteilhafter Weise vermieden.
- Auch ist vorteilhaft eine sichtbare Darstellung des Messzyklus vorgesehen. Daran kann ein automatisches Aufspüren des Zeitpunktes, an dem die Messung begonnen wurde, durch Auswertung der Venengeometrie und ihrer Verformung erfolgen (Beispielsweise Beginn der Messung bei 5% Geometrieveränderung von Venenhöhe zu Venenbreite).
   Ferner kann bevorzugt eine Benachrichtigung des Untersuchers über die Tatsache erfolgen, dass die Messung begonnen hat. Zum Beispiel kann dies durch Wechseln der Farben von bestimmten Bereichen des Bildschirms bzw. der Anzeigeeinheit geschehen. Vorteilhaft wird so sichergestellt, dass der Untersucher weiss, dass ein Messzyklus begonnen wurde, und ferner wird dem Untersucher ein weiteres Datum zur Auswertung in Form des Ausgangsdrucks bei der ersten Bewegung der Venengeometrie zur Verfügung gestellt.
- Auch ist eine sichtbare Darstellung des Endes des Messzyklus vorgesehen. Dies gestattet ein automatisches Aufspüren des Zeitpunktes, an dem die Vene 16 kollabiert ist und die Messung vervollständigt wurde, durch Auswertung der Venengeometrie und ihrer Verformung. Zum Beispiel kann "Beenden" vorzugsweise definiert sein, wenn 95% der Geometrieveränderung von Venenhöhe zu Venenbreite vorliegen, oder sobald kein hohler Bereich in der Vene 16 mehr detektiert werden kann.
   Bevorzugt erfolgt eine Benachrichtigung des Untersuchers über die Tatsache, dass die Messung abgeschlossen wurde. Dies geschieht zum Beispiel durch Wechseln der Farben von bestimmten Bereichen auf dem Bildschirm und durch Anzeige des Messergebnisses in, vorteilhaft grossen, Ziffern auf der Anzeigeeinheit (Bildschirm). Vorteilhaft wird so sichergestellt, dass der Untersucher weiss, dass ein Messzyklus beendet ist. Diese Daten können verwendet werden und ebenso kann eine neue Messung begonnen werden.
- Vorzugsweise kann erfindungsgemäss sichergestellt werden, dass die Fortführung der Messung durch weitere Datensammlung an derselben Position erfolgt. Daher erfolgt bevorzugt eine automatische Detektion von weiteren Messungen, die es gestattet, dem Untersucher nach einer schlechten Messung eine weitere Messung unter Berücksichtigung der oben erwähnten Kriterien durchzuführen. Falls der Messzyklus abgeschlossen ist, wird die neue Messung ebenso angezeigt. Bevorzugt wird ein Mittelwert mit den vorangehenden Messungen am gleichen Ort ebenso angezeigt mit dem Wert der vorangehenden Messung.
   Ein fortwährendes Überwachen des Drucks und des analysierten Bildes resultieren vorteilhaft in einer automatischen Detektion des Messendes. Sobald zum Beispiel der Untersucher die Druckmessvorrichtung von der Haut des Patienten abhebt oder sie zu weit vom Ausgangsort der Messung entfernt bzw. verschiebt, kann vorteilhaft die Messung beendet werden. Fallweise kann danach vorteilhaft ein neuer Messzyklus angestossen werden, sobald der Untersucher den Betätigungsknopf an der Druckmessvorrichtung drückt oder die Druckmessvorrichtung mit der Haut in Berührung kommt und der Druck ansteigt, wobei vorteilhaft Gewebe 4 durch eine Auswertungssoftware detektiert werden kann. Eine solche Option kann beispielsweise von einem persönlichen Einstellmenü innerhalb der Software ausgewählt werden.
- Um den Untersucher nicht zu verwirren und zum Zwecke einer übersichtliche Darstellung, werden vorteilhaft die gemessenen und ausgewerteten Daten in einem kombinierten Rahmen zusammen mit dem Ultraschallbild angezeigt, das durch die medizinische Ultraschallvorrichtung abgegeben wird.
- Um einem Untersucher aktuelle Informationen über die Qualität des Messzyklus zu geben, sollten die detektierten Typologien von Haut, Oberfläche der Venendruckvorrichtung, Vene, Knochen; wie oben dargelegt, auf dem Bildschirm angezeigt werden und farblich gemäss ihrem Status eingefärbt werden. Beispielsweise bedeutet Topologie grün "Kein Problem", rot "nicht akzeptabel" und orange "kritisch".

Das erfindungsgemässe Verfahren zur Venendruckmessung gibt eine einfache Vorgehensweise an, womit eine aus Druckmessvorrichtung und Ultraschallmesseinheit bestehende Venendruckmessvorrichtung zum automatisierten Messen von Venen- und/oder Organdrücken eingesetzt werden kann.

Für den Fachmann sind angesichts der voranstehenden Beschreibung und Ausführungsbeispielen der erfindungsgemässen Druckmessvorrichtung des Systems zur Venendruckmessung und des Verfahrens zur Venendruckmessung Abwandlungen im Rahmen der technischen Möglichkeiten und der bekannten Vorrichtungen zur Druck- und Ultraschallmessungen denkbar.

**Bezugszeichenliste**

| | | | |
|---|---|---|---|
| 10 | Körperteil/Unterarm | 210 | Gehäuseeinheit |
| | | 220 | Datenkabel |
| 11 | Ultraschalltransparente Flüssigkeit | 230 | Funktionstasten |
| | | 240 | Funktionstasten |
| 14 | Gewebe | 250 | Steckverbinder |
| | | | |
| 16 | Vene | | |
| 17 | Gel | 300 | Druckmessvorrichtung |
| 18 | Knochen | 305 | Gehäuse |
| | | 315 | 1. Gehäuseabschnitt |
| | | 316 | Aufnahme für 170 |
| | | 318 | Kontaktbereich v. 315 |
| 100 | Druckmessvorrichtung | 320 | Festlegeabschnitt |
| 105 | Gehäuse | 321 | Klettverschluss |
| 110 | Extremität | | |
| 115 | 1. Gehäuseabschnitt | | |
| 116 | Aufnahme für 170 | 500 | Druckmessvorrichtung |
| 120 | 2. Gehäuseteil | 505 | Gehäuse |
| 125 | Scharnier | 515 | 1. Gehäuseabschnitt |
| 130 | Anzeigeeinheit | 525 | Drucksensor |
| 135 | Verriegelungseinrichtung | 530 | Anzeigeeinheit |
| 140 | Funktionstasten | 550 | Druckerzeugungseinrichtung |
| 150 | Funktionstasten | 560 | Ansteuerungseinheit |
| 160 | Funktionstasten | 570 | Recheneinheit |
| 170 | Behältnis | | |
| 171 | Auflageseite v. 170 | | |
| 172 | Anlageseite v. 170 | 600 | Druckmessvorrichtung |
| 175 | Leitung zur Druckmessung | 615 | Sender |
| 180 | Fixierungsbehältnis | 620 | Empfänger |
| 190 | Ultraschallmesseinheit | | |
| 195 | Verbindungskabel | | |
| 200 | Halteeinrichtung | | |

## Patentansprüche

1. Druckmessvorrichtung (100; 300; 500; 600) zur Druckmessung einer Vene (16) oder eines Organs und zur Kombination mit einer Ultraschallmesseinheit (190) aufweisend:
ein Gehäuseabschnitt (115; 315; 515), ein Festlegeabschnitt (120; 320) zum Festlegen der Druckmessvorrichtung (100; 300; 500; 600) an einem Körperteil (10) und ein an dem Gehäuseabschnitt (115; 315; 515) vorgesehenes Behältnis (170), wobei das Behältnis (170) mit einer ultraschalltransparenten Flüssigkeit (11) gefüllt und zumindest eine mit der Oberfläche (15) eines Gewebes (14) des Körperteils in Anlage kommende Auflageseite (171) sowie eine der Auflageseite (171) gegenüberliegende Anlageseite (172) für die Anlage der Ultraschallmesseinheit (190) an dem Behältnis (170) aus einem ultraschalldurchlässigen Material gefertigt ist, wobei vorteilhaft wenigstens die Auflageseite (171) flexibel ist, um sich der Oberfläche (15) eines Gewebes (14) des Körperteils (10) anpassen zu können, wobei das Behältnis (170) so ausgestaltet ist, dass ein von der Ultraschallmesseinheit (190) ausgehender Ultraschall durch die Anlageseite (172) und die Auflageseite (171) durchtritt sowie vom Gewebe (14) reflektierter Ultraschall ebenso durch die Auflageseite (171) und die Anlageseite (172) zurück zur Ultraschallmesseinheit (190) durchtritt, wobei das Behältnis (170) ein abgeschlossenes Volumen der ultraschalltransparenten Flüssigkeit (11) umschliesst und die Flüssigkeit (11) im Behältnis (170) druckbeaufschlagbar ist.

2. Druckmessvorrichtung nach Anspruch 1, **dadurch gekennzeichnet, dass** der Festlegeabschnitt von wenigstens einem weiteren, zweiten Gehäuseteil (120) gebildet ist, das verschwenkbar und/oder verdrehbar an dem anderen Gehäuseabschnitt (115; 315; 515) angeordnet ist, wobei der Gehäuseabschnitt (115; 315; 515) und das wenigstens eine Gehäuseteil (120) vorteilhaft eine um das Körperteil (10) herum anordnenbare Manschette ausbilden.

3. Druckmessvorrichtung nach Anspruch 2, **dadurch gekennzeichnet, dass** zumindest ein Fixierungsbehältnis (180) an dem wenigstens einen Gehäuseteil (120) und/oder benachbart zu dem Behältnis (170) mit der ultraschalltransparenten Flüssigkeit (11) vorgesehen ist.

4. Druckmessvorrichtung nach einem der vorangehenden Ansprüche, **dadurch gekennzeichnet, dass** in dem Gehäuseabschnitt (115; 315; 515) eine Aufnahme (116; 316) zum zumindest bereichsweisen Aufnehmen des Behältnisses (170) vorgesehen ist.

5. Druckmessvorrichtung nach einem der vorangehenden Ansprüche, **dadurch gekennzeichnet, dass** ein Drucksensor (525), vorzugsweise in dem Gehäuseabschnitt (515), zur Messung des Druckes in der ultraschalltransparenten Flüssigkeit (11) vorgesehen ist.

6. Druckmessvorrichtung nach einem der vorangehenden Ansprüche, **dadurch gekennzeichnet, dass** eine Druckerzeugungseinrichtung (550), vorzugsweise in dem Gehäuseabschnitt (515), zur Druckbeaufschlagung zumindest der ultraschalltransparenten Flüssigkeit (11) in dem Behältnis (170) vorgesehen ist.

7. Druckmessvorrichtung nach einem der vorangehenden Ansprüche, **dadurch gekennzeichnet, dass** eine Ansteuerungseinheit (560), vorzugsweise in dem Gehäuseabschnitt (515), zur Ansteuerung einer zu messenden Vene (16) bzw. eines zu messenden Organs vorgesehen ist.

8. Druckmessvorrichtung nach Anspruch 5 bis 7, **dadurch gekennzeichnet, dass** ein Verbindungskabel zur Weitergabe der vom Drucksensor (525) ermittelten Werte vorgesehen ist oder dass ein Sender (615) zur Weitergabe der vom Drucksensor (525) ermittelten Werte vorgesehen ist.

9. Druckmessvorrichtung nach einem der vorangehenden Ansprüche, **dadurch gekennzeichnet, dass** eine Weiterverarbeitungseinheit, vorzugsweise in dem Gehäuseabschnitt (515), zur Weiterverarbeitung von ermittelten Werten des Drucksensors (525) vorgesehen ist.

10. Druckmessvorrichtung nach einem der vorangehenden Ansprüche, **dadurch gekennzeichnet, dass** eine Anzeigeeinheit (130; 530) zur Anzeige von ermittelten Werten des Drucksensors (525) vorgesehen ist, wobei vorteilhaft die Anzeigeeinheit (130) in einer separaten Gehäuseeinheit (210) angeordnet ist, wobei die Gehäuseeinheit (210) zumindest eine Auswertevorrichtung und/oder Verarbeitungseinheit und/oder Funktionstasten (140, 150, 160, 230, 240) zur Steuerung der Druckmessvorrichtung aufweist.

11. Druckmessvorrichtung nach einem der vorangehenden Ansprüche, **dadurch gekennzeichnet, dass** eine Halterungseinrichtung (200) für die Ultraschallmesseinheit (190) an oder im Gehäuseabschnitt (115) vorgesehen ist oder die Ultraschallmesseinheit (190) Bestandteil der Druckmessvorrichtung (100; 300; 500; 600) ist.

12. Druckmesssystem zur Druckmessung einer Vene (16) oder eines Organs wenigstens aufweisend:
eine Druckmessvorrichtung (100; 300; 500; 600) nach einem der Ansprüche 1 bis 11 und eine Ultraschallmesseinheit (190), wobei die Druckmessvorrichtung (100; 300; 500; 600) und die Ultraschallmesseinheit (190) miteinander vorteilhaft mechanisch zu einer Messeinheit gekoppelt sind oder zu einer Messeinheit verbunden sind.

13. Verfahren zur Druckmessung einer Vene (16) oder eines Organs bei dem ein Druckmesssystem (100; 300; 500; 600) nach Anspruch 12 um ein Körperteil (10), unter dem sich eine zu messende Vene (16) bzw. das zu messende Organ befindet, herumgelegt sowie vorteilhaft an diesem fixiert wird, solange der Druck im Behältnis (170) und somit auf das Gewebe (14) gesteigert wird, bis sich aus der Ultraschallmessung ergibt, dass die Vene (16) kollabiert bzw. das Organ einen vorbestimmten Verformungsgrad erreicht, wobei das von der Ultraschallmesseinheit (190) abgegebene Signal dazu verwendet wird, den Zeitpunkt einer Druckmessung festzulegen und wobei der zu diesem Zeitpunkt anliegende Druck von der Druckmessvorrichtung (100; 300; 500; 600) als Venendruck bzw. Organdruck bestimmt wird, wobei die Auswertung von Messinformation vorzugsweise zumindest teilweise im Bereich eines Drucksensors (525) erfolgt und ausgewertete Messinformationen über Funk (615, 620) oder Verbindungskabel an eine Anzeigeeinheit (130; 530) und/oder Auswerteeinheit weitergeleitet werden.

14. Verfahren zur Druckmessung einer Vene oder eines Organs nach Anspruch 13, **dadurch gekennzeichnet, dass** der Zeitpunkt der Druckmessung in einer Verarbeitungseinheit automatisch ermittelt wird.

15. Verfahren zur Druckmessung einer Vene oder eines Organs nach Anspruch 13 oder 14, **dadurch gekennzeichnet, dass** der Zeitpunkt des Kollabierens einer Vene (16) oder das Erreichen eines bestimmten Verformungsgrades eines Organs über ein bildverarbeitendes Verfahren bestimmt wird.

## Claims

1. A pressure measuring device (100; 300; 500; 600) for measuring the pressure of a vein (16) or an organ and for combination with an ultrasound measurement unit (190), comprising: a housing section (115; 315; 515), an immobilizing section (120; 320) for immobilizing the pressure measuring device (100; 300; 500; 600) on a body part (10) and a container (170) provided on the housing section (115; 315; 515), wherein the container (170) is filled with liquid (11) which is transparent to ultrasound and at least a placement side (171) which will be placed on the surface (15) of tissue (14) of the body part as well as an attachment side (172) opposite to the placement side (171) for the attachment of the ultrasound measurement unit (190) to the container (170) is produced from a material which is transparent to ultrasound, wherein advantageously, at least the placement side (171) is flexible, so that it can conform to the surface (15) of tissue (14) of the body part (10), wherein the container (170) is configured in a manner such that ultrasound emanating from the ultrasound measurement unit (190) penetrates through the attachment side (172) and the placement side (171), and also ultrasound reflected from the tissue (14) similarly penetrates through the placement side (171) and the attachment side (172) back to the ultrasound measurement unit (190), wherein the container (170) encloses a closed volume of the liquid (11) which is transparent to ultrasound and the liquid (11) in the container (170) can be pressurized.

2. The pressure measuring device as claimed in claim 1, **characterized in that** the immobilizing section is formed from at least one further, second housing part (120) which is disposed in a pivotable and/or rotatable manner on the other housing section (115; 315; 515), wherein the housing section (115; 315; 515) and the at least one housing part (120) advantageously form a cuff which can be disposed around the body part (10).

3. The pressure measuring device as claimed in claim 2, **characterized in that** at least one fixing container (180) is provided on the at least one housing part (120) and/or adjacent to the container (170) with the liquid (11) which is transparent to ultrasound.

4. The pressure measuring device as claimed in one of the preceding claims, **characterized in that** a receptacle (116; 316) is provided in the housing section (115; 315; 515) to accommodate at least regions of the container (170).

5. The pressure measuring device as claimed in one of the preceding claims, **characterized in that** a pressure sensor (525), preferably in the housing section (515), is provided for measuring the pressure in the liquid (11) which is transparent to ultrasound.

6. The pressure measuring device as claimed in one of the preceding claims, **characterized in that** a pressure producing device (550), preferably in the housing section (515), is provided to pressurize at least the liquid (11) which is transparent to ultrasound in the container (170).

7. The pressure measuring device as claimed in one of the preceding claims, **characterized in that** a control unit (560), preferably in the housing section (515), is provided for controlling a vein (16) to be measured or an organ to be measured.

8. The pressure measuring device as claimed in claims 5 to 7, **characterized in that** a connecting cable is provided for transmitting the values detected by the pressure sensor (525), or **in that** a transmitter (615) is provided for transmitting the values detected by the pressure sensor (525).

9. The pressure measuring device as claimed in one of the preceding claims, **characterized in that** a processing unit is provided, preferably in the housing section (515), in order to process values detected by the pressure sensor (525).

10. The pressure measuring device as claimed in one of the preceding claims, **characterized in that** a display unit (130; 530) is provided in order to display the values detected by the pressure sensor (525), wherein advantageously, the display unit (130) is disposed in a separate housing unit (210), wherein the housing unit (210) comprises at least one evaluation device and/or processing unit and/or function key (140, 150, 160, 230, 240) for controlling the pressure measuring device.

11. The pressure measuring device as claimed in one of the preceding claims, **characterized in that** a retaining device (200) for the ultrasound measurement unit (190) is provided on or in the housing section (115) or the ultrasound measurement unit (190) is a component of the pressure measuring device (100; 300; 500; 600).

12. A pressure measuring system for measuring the pressure of a vein (16) or an organ, comprising at least:
a pressure measuring device (100; 300; 500; 600) as claimed in one of claims 1 to 11 and an ultrasound measurement unit (190), wherein the pressure measuring device (100; 300; 500; 600) and the ultrasound measurement unit (190) are coupled together, advantageously mechanically, to form a measurement unit or are connected to form a measurement unit.

13. A method for measuring the pressure of a vein (16) or an organ, in which a pressure measuring system (100; 300; 500; 600) as claimed in claim 12 is placed around a body part (10) below which the vein (16) to be measured or the organ to be measured is located, and advantageously fixed thereto, for as long as the pressure in the container (170) and therefore on the tissue (14) is increased until the ultrasound measurement indicates that the vein (16) has collapsed or the organ has reached a predetermined degree of deformation, wherein the signal emitted by the ultrasound measurement unit (190) is used to establish the time of a pressure measurement and wherein the pressure prevailing at this time is determined by the pressure measuring device (100; 300; 500; 600) as the venous pressure or the organ pressure, wherein the evaluation of measurement information is advantageously carried out at least in part in the region of a pressure sensor (525) and evaluated measurement information is transmitted wirelessly (615, 620) or via a connecting cable to a display unit (130; 530) and/or an evaluation unit.

14. The method for measuring the pressure of a vein or an organ as claimed in claim 13, **characterized in that** the time of the pressure measurement is automatically ascertained in a processing unit.

15. The method for measuring the pressure of a vein or an organ as claimed in claim 13 or claim 14, **characterized in that** the time for the collapse of a vein (16) or for reaching a specified degree of deformation of an organ is determined by means of an image processing method.

## Revendications

1. Dispositif de mesure de pression (100 ; 300 ; 500 ; 600) destiné à mesurer la pression d'une veine (16) ou d'un organe et à être associé avec une unité de mesure aux ultrasons (190), comportant :
une partie boîtier (115 ; 315 ; 515), une partie immobilisation (120 ; 320), destiné à immobiliser le dispositif de mesure de pression (100 ; 300 ; 500 ; 600) sur une partie du corps (10) et un contenant (170) prévu sur la partie boîtier (115 ; 315 ; 515), le contenant (170) étant rempli d'un liquide transparent aux ultrasons (11) et au moins une face d'appui (171) entrant en appui avec la surface (15) d'un tissu (14) de la partie du corps, ainsi qu'une face de pose (172) opposée à la face d'appui (171) destinée à poser l'unité de mesure aux ultrasons (190) sur le contenant (170) et étant fabriqué en une matière transmettant les ultrasons, de préférence, au moins la face d'appui (171) étant souple, pour être apte à s'adapter à une surface (15) d'un tissu (14) de la partie du corps (10), le contenant (170) étant conçu de sorte qu'un ultrason partant de l'unité de mesure aux ultrasons (190) traverse la face de pose (172) et la face d'appui (171) et qu'un ultrason réfléchi par le tissu (14) traverse également la face d'appui (171) et la face de pose (172), en retour vers l'unité de mesure aux ultrasons (190), le contenant (170) entourant un volume clos du liquide transparent aux ultrasons (11) et le liquide (11) étant susceptible d'être soumis à une pression dans le contenant (170).

2. Dispositif de mesure de pression selon la revendication 1, **caractérisé en ce que** la partie immobilisation est formée d'au moins une deuxième partie boîtier (120) supplémentaire, qui est placée de sorte à pouvoir pivoter et/ou tourner sur l'autre partie boîtier (115 ; 315 ; 515), la partie boîtier (115 ; 315 ; 515) et l'au moins une partie boîtier (120) formant avantageusement une manchette susceptible d'être placée autour de la partie du corps (10) .

3. Dispositif de mesure de pression selon la revendication 2, **caractérisé en ce qu'**au moins un contenant de fixation (180) est prévu sur l'au moins une partie boîtier (120) et/ou au voisinage du contenant (170) avec le liquide transparent aux ultrasons (11).

4. Dispositif de mesure de pression selon l'une quelconque des revendications précédentes, **caractérisé en ce que** dans la partie boîtier (115 ; 315 ; 515) est prévu un logement (116 ; 316), destiné à recevoir au moins par endroits le contenant (170).

5. Dispositif de mesure de pression selon l'une quelconque des revendications précédentes, **caractérisé en ce qu'**un capteur de pression (525) est prévu de préférence dans la partie boîtier (515), pour mesurer la pression dans le liquide transparent aux ultrasons (11) .

6. Dispositif de mesure de pression selon l'une quelconque des revendications précédentes, **caractérisé en ce qu'**un générateur de pression (550) est prévu de préférence dans la partie boîtier (515), pour soumettre à la pression au moins le liquide transparent aux ultrasons (11) dans le contenant (170) .

7. Dispositif de mesure de pression selon l'une quelconque des revendications précédentes, **caractérisé en ce qu'**une unité d'activation (560) est prévue de préférence dans la partie boîtier (515), pour activer une veine (16) qui doit être mesurée ou un organe qui doit être mesuré.

8. Dispositif de mesure de pression selon la revendication 5 à 7, **caractérisé en ce qu'**un câble de connexion est prévu pour retransmettre les valeurs déterminées par le capteur de pression (525) ou **en ce qu'**un émetteur (615) est prévu pour retransmettre les valeurs déterminées par le capteur de pression (525).

9. Dispositif de mesure de pression selon l'une quelconque des revendications précédentes, **caractérisé en ce qu'**une unité de traitement ultérieur est prévue, de préférence dans la partie boîtier (515), pour le traitement ultérieur de valeurs déterminées par le capteur de pression (525).

10. Dispositif de mesure de pression selon l'une quelconque des revendications précédentes, **caractérisé en ce qu'**une unité d'affichage (130 ; 530) est prévue, pour afficher des valeurs déterminées par le capteur de pression (525), l'unité d'affichage (130) étant placée avantageusement dans une unité de boîtier (210) séparée, l'unité de boîtier (210) comportant au moins un dispositif d'évaluation et/ou une unité de traitement et/ou des touches de fonction (140, 150, 160, 230, 240) pour commander le dispositif de mesure de pression.

11. Dispositif de mesure de pression selon l'une quelconque des revendications précédentes, **caractérisé en ce qu'**un système d'attache (200) est prévu pour l'unité de mesure aux ultrasons (190) sur ou dans la partie boîtier (115) ou **en ce que** l'unité de mesure aux ultrasons (190) est une partie intégrante du dispositif de mesure de pression (100 ; 300 ; 500 ; 600) .

12. Système de mesure de la pression, destiné à mesurer la pression d'une veine (16) ou d'un organe, comportant au moins :
un dispositif de mesure de pression (100 ; 300 ; 500 ; 600) selon l'une quelconque des revendications 1 à 11 et une unité de mesure aux ultrasons (190), le dispositif de mesure de pression (100 ; 300 ; 500 ; 600) et l'unité de mesure aux ultrasons (190) étant accouplés l'un à l'autre, avantageusement de manière mécanique en une unité de mesure ou étant assemblés en une unité de mesure.

13. Procédé, destiné à mesurer la pression d'une veine (16) ou d'un organe, lors duquel on pose un système de mesure de la pression (100 ; 300 ; 500 ; 600) selon la revendication 12 autour d'une partie du corps (10), sous laquelle se trouve une veine (16) qui doit être mesurée ou l'organe qui doit être mesuré et on l'y fixe avantageusement, aussi longtemps que la pression dans le contenant (170) et donc exercée sur le tissu (14) augmente, jusqu'à ce qu'il résulte de la mesure aux ultrasons que la veine (16) s'affaisse ou que l'organe atteint un certain niveau de déformation, le signal délivré par l'unité de mesure aux ultrasons (190) étant utilisé pour déterminer le moment d'une mesure de la pression et la pression appliquée audit moment étant définie par le dispositif de mesure de pression (100 ; 300 ; 500 ; 600) comme étant la pression veineuse ou la pression de l'organe, l'évaluation de l'information de mesure s'effectuant de préférence au moins en partie dans la zone d'un capteur de pression (525) et des informations de mesure évaluées étant retransmises par radio (615, 620) ou par des câbles de connexion à une unité d'affichage (130 ; 530) et/ou à une unité d'évaluation.

14. Procédé, destiné à mesurer la pression d'une veine ou d'un organe selon la revendication 13, **caractérisé en ce que** le moment de la mesure de la pression est déterminé automatiquement dans une unité de traitement.

15. Procédé, destiné à mesurer la pression d'une veine ou d'un organe selon la revendication 13 ou 14, **caractérisé en ce que** le moment de l'affaissement d'une veine (16) ou l'atteinte d'un certain niveau de déformation d'un organe est déterminé(e) par un procédé de traitement d'images.
